# EUROPÄISCHE PATENTSCHRIFT

(11) **EP 2 809 265 B1**
(45) Veröffentlichungstag und Bekanntmachung des Hinweises auf die Patenterteilung: **14.09.2016**
(21) Anmeldenummer: 13702975.7
(22) Anmeldetag: 29.01.2013
(51) Int. Cl.: A61F 2/07

(54) **MODULARER STENTGRAFT**
MODULAR STENT GRAFT
ENDOPROTHÈSE MODULAIRE

(30) Priorität: 31.01.2012 DE 102012100754
(43) Veröffentlichungstag der Anmeldung: 10.12.2014
(73) Patentinhaber: JOTEC GmbH, 72379 Hechingen (DE)
(72) Erfinder: WOERNE, Christian, 73760 Ostfildern (DE)
(74) Vertreter: Witte, Weller & Partner Patentanwälte mbB
(86) Internationale Anmeldenummer: PCT/EP2013/051697
(87) Internationale Veröffentlichungsnummer: WO 2013/113704

(56) Entgegenhaltungen:
- WO-A1-2006/028925
- US-A- 5 824 037
- US-A1- 2005 010 277

## Beschreibung

Die vorliegende Erfindung betrifft einen modularen Stentgraft aus zwei Stentgraft-Modulen zur Einführung in ein Blutgefäß eines Patienten.

Unter Stentgrafts werden im Allgemeinen endoluminale Prothesen bzw. intravaskuläre Implantate verstanden, die ihren Einsatz bei geschwächten, verletzten, gerissenen oder aneurysmatischen Gefäßen finden. Hierfür wird an der erkrankten oder verletzten Stelle des Gefäßes ein Stentgraft freigesetzt, der die Funktionalität des ursprünglichen Gefäßes wieder herstellt bzw. die noch bestehende Gefäßintegrität zu unterstützen. Der Stentgraft, der radial expandierbar ist, wird hierzu in das betreffende Gefäß eingeführt, und radial vergrößert oder expandiert.

Stentgrafts bestehen allgemein aus einem röhrenförmigen oder tubulären, d.h. hohlzylindrischen Grundkörper aus einem biokompatiblen Graft-Material, an das ein oder mehrere Stents oder Stentringe angebracht sind. Üblicherweise sind die bei den Stentgrafts derzeit verwendeten Stents selbstexpandierend, und umfassen ein sogenanntes Shape-Memory-Material, bspw. Nitinol, durch welches sich der Stentgraft insgesamt von seinem nicht-komprimierten Zustand in einen komprimierten Zustand überführen und anschließend, d.h. nach Freisetzung, wieder in den nicht-komprimierten Zustand expandieren lässt. Zur Einführung in das zu behandelnde Blutgefäß wird der Stentgraft also komprimiert und im komprimierten Zustand in das Gefäß vorgeschoben, bis er an der gewünschten Stelle zu liegen kommt. Dort wird er freigesetzt, d.h. ihm wird die Überführung und Expansion in den nicht-komprimierten Zustand ermöglicht. Die Wände des Stentgrafts legen sich dabei an die Gefäßwand an und ersetzen und/oder unterstützen diese dadurch. Auf diese Weise wird der Defekt verschlossen und das in dem Gefäß geführte Blut kann durch den röhrenförmigen Grundkörper des Stentgrafts fließen.

Stentgrafts bzw. Gefäßimplantate können allgemein aus einem einzigen Stentgraft-Körper bestehen, oder alternativ aus mehreren getrennten, aber zusammensetzbaren Stentgraft-Körpern bzw. Stentgraft-Modulen. Dabei bietet ein modular aufgebauter Stentgraft den Vorteil, dass er diesen speziell auf das mit dem Stentgraft zu behandelnde Gefäß/Gefäßbereich und dessen unterschiedliche Durchmesser oder Längen zusammengestellt werden kann. Die einzelnen, getrennten Module des Stentgrafts werden dann in der Regel in situ, also im Gefäß an der zu behandelnden Stelle zur Ausbildung eines zusammengesetzten Stentgrafts ein- und zusammengesetzt. Hierfür wird üblicherweise ausgenutzt, dass sich die Enden der einzelnen Stentgraft-Module überlappen, und das eine Ende eines Stentgraft-Moduls in ein Ende eines anderen Stentgraft-Moduls überlappend eingesetzt werden kann. Aufgrund der selbstexpandierenden, nach außen und in Richtung Gefäßwand wirkenden Kraft bleiben die Module dabei über Reibungskräfte in den überlappenden Bereichen miteinander verbunden.

Aufgrund der besonderen Beschaffenheit der jeweils zu behandelnden Gefäße werden in vielen Fällen speziell auf den Patienten bzw. dessen Gefäße, und insbesondere auf das speziell zu behandelnde Gefäß zugeschnittene Prothesen bereitgestellt, um die Anforderungen hinsichtlich der erforderlichen bzw. erwünschten Länge und des Durchmessers nachzukommen. Diese spezielle und spezifische Anfertigung ist aufwändig, teuer sowie arbeits- und kostenintensiv, da jeweils nur eine spezifische Prothese gefertigt werden kann.

Grundsätzlich besteht daher nach wie vor ein großes Bedürfnis an Prothesen, die einen flexiblen Einsatz ermöglichen, um den unterschiedlichen Anforderungen - insbesondere im Hinblick auf Länge und Durchmesser- der jeweiligen Gefäße verschiedener Patienten nachzukommen. Aufgabe der vorliegenden Erfindung ist es daher, ein alternatives Prothesensystem bereitzustellen, das ein maßgeschneidertes System, insbesondere im Hinblick auf die Gefäßlänge und den Durchmesser, überflüssig macht.

Erfindungsgemäß wird diese Aufgabe dadurch gelöst, dass ein modularer Stentgraft bereitgestellt wird, der zur Einbringung in ein Blutgefäß an einer Stelle ohne Blutgefäß-Verzweigung eines Patienten ausgebildet ist, und der über ein Graftmaterial verbundene Stents aufweist, wobei der Stentgraft folgendes aufweist: einen ersten Stentgraft-Modul mit einem Rumpf, einem proximalen und einem distalen Ende, sowie mit zumindest zwei bis vier sich in Längsrichtung vom distalen Ende des ersten Stentgraft-Moduls aus in distale Richtung erstreckenden röhrenförmigen Ärmchen, die einstückig mit dem Rumpf des ersten Stentgraft-Moduls verbunden sind; einen zweiten Stentgraft-Modul mit einem zweiten Rumpf, einem proximalen und einem distalen Ende, sowie mit zumindest drei sich in Längsrichtung vom proximalen Ende des zweiten Stentgraft-Moduls in proximale Richtung erstreckenden röhrenförmigen Ärmchen, die einstückig mit dem Rumpf des zweiten Stentgraft-Moduls ausgebildet sind; dabei weisen jeweils ein Ärmchen des ersten und des zweiten Stentgraft-Moduls Durchmesser auf, derart, dass der erste und zweite Stentgraft-Modul durch ein Ineinanderschieben und durch teilweises Überlappen des jeweils einen Ärmchens des distalen Endes des ersten Stentgraft-Moduls und des Ärmchens des proximalen Endes des zweiten Stentgraft-Moduls zur Ausbildung eines zusammengesetzten modularen Stentgrafts im gleichen Blutgefäß miteinander verbindbar sind.

Die der Erfindung zugrunde liegende Aufgabe wird dadurch vollkommen gelöst.

Vorliegend wird - wie allgemein im Stand der Technik - unter einem "Stentgraft-Modul" ein einzelnes Stentgraft-Element eines Stentgrafts bzw. Stentgrafts-System verstanden, das im nicht implantierten bzw. nicht in ein Gefäß eingeführten Zustand die einzelnen, separaten und nicht miteinander verbundenen Stentgraft-Module umfasst, und das nach Einbringung der einzelnen Stentgraft-Elemente in ein Gefäß in diesem einen zusammengesetzten Stentgraft/Stentgraft-System aus den Stentgraft-Elementen bildet. Die Stentgraft-Elemente bzw. -Module werden dabei durch Überlappende An- und Einbringung der Enden der jeweiligen Stentgraft-Module miteinander verbunden und bleiben durch reibende Haftung und durch die radiale Expansionskraft der Stentelemente der einzelnen Stentgraft-Module miteinander sozusagen indirekt verbunden bzw. verankert.

Vorliegend wird ferner unter einem "Stentgraft" bzw. "Stentgraft-Modul" ein einen röhrenförmigen oder tubulären, d.h. hohlzylindrischen Grundkörper umfassendes Gefäßimplantat bzw. Gefäßimplantat-Modul verstanden, mit einem biokompatiblen Graft-Material, an das ein oder mehrere selbstexpandierende Stents bzw. Stentringe angebracht sind. Über die radial selbstexpandierenden Stentringe bzw. Stents kann der Stentgraft bzw. der Stentgraft-Modul zur Einbringung in ein Gefäß eines Patienten von seinem ursprünglich nicht-komprimierten Zustand in einen komprimierten Zustand überführt werden und anschließend, d.h. nach Freisetzung, wieder in den nicht-komprimierten Zustand expandieren. Zur Einführung in das zu behandelnde Blutgefäß wird der Stentgraft bzw. Stentgraft-Modul also komprimiert und im komprimierten Zustand in das Gefäß vorgeschoben, bis er an der gewünschten, zu behandelnden Stelle zu liegen kommt. Dort wird er freigesetzt, d.h. ihm wird die Überführung und Expansion in den nicht-komprimierten Zustand ermöglicht. Die Wände des Stentgrafts/Stentgraft-Moduls legen sich dabei an die Gefäßwand an und ersetzen und/oder unterstützen diese dadurch. Auf diese Weise wird der Defekt verschlossen und das in dem Gefäß geführte Blut kann durch den röhrenförmigen Grundkörper des Stentgrafts fließen.

Vorliegend und durchgehend durch die Beschreibung wird der erfindungsgemäße modulare Stentgraft auch synonym mit "modulares Stentgraftsystem" oder "modulare Stentgraft-Baugruppe" bezeichnet.

Modulare Stentgrafts werden gegenwärtig üblicherweise an Stellen in Blutgefäßen eingesetzt, an welchen Seitengefäße abzweigen. Die hierbei eingesetzten Prothesen weisen üblicherweise Seitenäste auf, die in den abzweigenden Gefäßen zu liegen kommen. Diese modularen Gefäßprothesen dienen dazu, auch an Verzweigungen von Gefäßen ein abdichtendes Implantat anzubringen, und um gleichzeitig zu verhindern, dass ein abzweigendes Gefäß blockiert wird.

So ist bspw. aus der WO 2000/076423 ein modulares Stentgraftsystem bzw. eine Baugruppe bekannt, die aus zwei rohrförmigen Prothesen besteht, die im Bereich der Verzweigung der abdominalen Aorta in die iliatrischen Arterien ein- bzw. zusammengesetzt werden.

Aus der US-A-.2005/0010277 ist ein weiterer modularer Stent-Graft bekannt.

Auch die WO 2006/028925 beschreibt ein modulares Prothesensystem, das einen ersten Modul mit einem Seitenast und einen zweiten Modul mit einer Kerbe aufweist; dieses System wird im Bereich des Aortenbogens eingesetzt, in dem verschiedene Gefäße abzweigen.

Aus der US2003/0120333 A1 ist eine Prothesensystem mit mehreren Modulen bekannt, mit einem Haupt-Modul mit einem Rumpf und mit sich in distale Richtung erstreckenden röhrenförmigen Ärmchen. An diese Ärmchen können jeweils separat Beinchen zur Ausbildung von in abzweigende Gefäße hineinreichenden Stentgraft-Ästen angebracht werden.

Ferner ist aus der US 5,824,037 ein modulares Prothesensystem bekannt, bei dem zwei oder mehr röhrchenförmige Prothesen über keilartig aufgeweitete Enden ineinandergesteckt werden können.

Die zuvor genannten Druckschriften beschreiben daher lediglich den Einsatz von modularen Stentgrafts bei Bifurkationen, mithin also bei Aufzweigungen von Blutgefäßen. Damit war allerdings nicht bekannt oder offenbart, modulare Stentgrafts im gleichen Gefäß einzusetzen. Hierbei wird unter einem Einsatz eines modularen Stentgrafts im "gleichen Blutgefäß" verstanden, dass der modulare Stentgraft nicht an einer Aufzweigung von Blutgefäßen zusammen- und eingesetzt wird, sondern an einer unverzeigten Stelle des Blutgefäßes, so dass die beiden Stentgraft-Module im gleichen Gefäß zu liegen kommen, und nicht der eine Stentgraft-Modul in dem Hauptgefäß und der andere Stentgraft-Modul in einem hiervon abzweigenden Gefäß.

Erfindungsgemäß wird dadurch erreicht, dass der Stentgraft einfacher eingeführt werden kann, da er in zwei Schritten zusammengesetzt wird, was ein flexibleres Freisetzen und eine einfachere Positionierung ermöglicht. Mit dem erfindungsgemäßen Stentgraft ist es möglich, eine korrekte Platzierung einfach zu erreichen, ohne dass es auf die spezifische Länge, Dimension und Ausbildung der Teile im Hinblick auf die Gefäßanatomie ankäme: So kann bspw. durch Rotation des einen oder des anderen Moduls im Gefäß die spezifisch gewünschte und erforderliche Position eingestellt werden. Dadurch ist der modulare Stentgraft flexibel einsetzbar, was nicht nur einen jeweils individuellen Einsatz gewährleistet, sondern auch eine breitere Anwendung für unterschiedliche Gefäße und Patienten ermöglicht.

Grundsätzlich werden bei Stentgrafts oder endoluminalen Prothesen allgemein sowie vorliegend zur Bezeichnung der jeweiligen Enden die Begriffe "distal" und "proximal" verwendet, wobei der Begriff "distal" der Teil bzw. das Ende bezeichnet wird, der/das in Bezug auf den Blutstrom weiter stromabwärts liegt. Der Begriff "proximal" hingegen bezeichnet, wieder in Bezug auf den Blutstrom, einen Teil bzw. das Ende, der/das in Bezug auf den Blutstrom weiter stromaufwärts liegt. Anders ausgedrückt bedeutet der Begriff "distal" in Richtung des Blutstroms, und der Begriff "proximal" der Richtung des Blutstroms entgegengesetzt. Bei Kathetern hingegen, bzw. Einführsystemen bezeichnet der Begriff "distal" das Ende des Katheters bzw. Einführsystems, das in den Patienten eingeführt wird, bzw. das vom Anwender ausgesehen am Entferntesten liegt, und der Begriff "proximal" das Ende, das sich dem Anwender näher zugewandt ist.

Beide Stentgraft-Module weisen Stents bzw. Stentringe auf, die auf der der Gefäßwand zugewandten Seite über ein biokompatibles Graft- oder Prothesenmaterial miteinander verbunden sind. Die Stents stellen mäanderförmig umlaufende Ringe aus einem selbstexpandierenden Material mit Shape-Memory dar, vorzugsweise Nitinol, die abwechselnd in proximale und distale Richtung weisende Spitzbögen oder Stützen aufweisen. Auch die Ärmchen der Stentgraft-Module sind auf diese Weise aufgebaut. Eine nähere Beschreibung solcher Stents sowie des Graft-/Prothesenmaterials findet sich bspw. in der DE 103 37 739.

Die bei der vorliegenden Erfindung eingesetzten Graftmaterialien weisen ein biokompatibles Material auf, und sind vorzugswiese ausgewählt aus Polyestern, wie bspw. Polyethylenterephthalate, Polylactid, Polyglycolid und Copolymeren hieraus; ferner aus Polytetrafluorethylen (PTFE), expandiertes PTFE (ePTFE) und Polyvinylidenfluorid; Polysiloxane; Polyurethane, etc. Darüber hinaus können auch Materialien eingesetzt werden, die nicht inhärent biokompatibel sind, die jedoch entsprechend behandelt wurden. Das Graftmaterial ist dabei bevorzugt ein gewebtes Material, das eines oder mehrere der zuvor genannten Materialien aufweist oder aus diesen besteht.

Erfindungsgemäß weist der erste Stentgraft-Modul dabei zumindest zwei bis vier, insbesondere drei, Ärmchen auf, die sich vom distalen Ende des Moduls in distale Längsrichtung, mithin also in Richtung des Blutflusses erstrecken. Durch diese Ärmchen können bspw. unterschiedliche bei der Einführung, Handhabung oder Nutzung des Stentgrafts erforderliche Vorrichtungen ein- und durchgeführt werden, wie bspw. Führungsdrähte, Katheter, Stents, Stentgrafts, etc. Über die Ärmchen wird eine Verbindung zu abgehenden Gefäßabschnitten, wie bspw. zu dem Truncus coeliacus und der Arteria mesentrica superior, hergestellt.

Auch der zweite Stentgraft-Modul weist Ärmchen auf, die sich an dessen proximalen Ende in proximale Richtung erstrecken, und damit den sich in distale Richtung erstreckenden Ärmchen des ersten Stentgraft-Moduls entgegen weisen. Die Ärmchen des zweiten Stentgraft-Moduls sind vorgesehen, um eine Verbindung zu abgehenden Gefäßabschnitten, wie bspw. den Arteriae renales, herzustellen.

Erfindungsgemäß ist dabei ist der Durchmesser eines Ärmchens des ersten Stentgraft-Moduls mit dem Durchmesser eines Ärmchens des zweiten Stentgraft-Moduls derart abgestimmt, dass diese ineinander geführt oder ineinander gesteckt werden können, so dass sich die Enden der Ärmchen in situ zumindest teilweise überlappen. Dadurch ist ein Teil des Endes des einen Stentgraft-Moduls im anderen Stentgraft-Modul relativ verankert. Diese teleskopartige Befestigung wird in der Regel durch Reibungskräfte in der Überlappungsregion erreicht, sowie durch die radiale Kraft, die der innen eingeführte Stentgraft-Modul auf den äußeren Stentgraft-Modul ausübt.

Je nach dem, welcher Stentgraft-Modul als erstes eingeführt bzw. gelegt wird, befindet sich entweder das distale Ende des Ärmchen des ersten Stentgraft-Moduls im proximalen Ende des zweiten Stentgraft-Moduls, oder umgekehrt das proximale Ende des Ärmchens des zweiten Stentgraft-Moduls im distalen Ende des Ärmchens des ersten Stentgraft-Moduls: Wird der erste, proximal zu liegen kommende Stentgraft-Modul zuerst eingeführt und platziert, wird das proximale Ärmchen des zweiten Stentgraft-Moduls in das distale Ende des Ärmchens des ersten Stentgraft-Moduls eingeführt; wird der zweite Stentgraft-Modul zuerst platziert, wird das distale Ende des Ärmchens des ersten Stent-graft-Moduls in das proximale Ende des Ärmchens des zweiten Stentgraft-Moduls eingeführt und dort verankert.

Dies ermöglicht eine bequeme, einfache und flexible Einbringung des modularen Stentgrafts in Abstimmung auf die jeweils vorliegenden Gefäßbedingungen.

Dementsprechend sind in einer bevorzugten Alternative des erfindungsgemäßen modularen Stentgrafts das erste und das zweite Stentgraft-Modul durch Ineinanderschieben eines Ärmchens des zweiten Stentgraft-Moduls in ein Ärmchen des ersten Stentgraft-Moduls und durch deren teilweises Überlappen verbindbar sind, wohingegen gemäß einer anderen bevorzugten Alternative das erste und das zweite Stentgraft-Modul durch Ineinanderschieben eines Ärmchens des ersten Stentgraft-Moduls in ein Ärmchen des zweiten Stentgraft-Moduls und durch deren teilweises Überlappen verbindbar sind.

Gemäß einer bevorzugten Ausführungsform weisen die Ärmchen des ersten und/oder zweiten Stentgraft-Moduls unterschiedliche Längen und/oder unterschiedliche Durchmesser auf. Beispielhaft können zwei Ärmchen des ersten Stentgraft-Moduls einen gleichen Durchmesser aufweisen, bspw. von jeweils ca. 8 mm, und ein Ärmchen einen Durchmesser von ca. 14 mm. "Ca. " bedeutet dabei, dass - wie im betreffenden Gebiet vollkommen verständlich - Mess- und Fertigungstoleranzen, die sich knapp ober- oder unterhalb der zuvor genannten und nachstehend noch aufgeführten Zahlenangaben befinden - wie bspw. 8,4 oder 14, 3 - noch im Bereich der Erfindung liegen.

Gemäß einer weiteren bevorzugten Ausführungsform weisen zwei Ärmchen des zweiten Stentgraft-Moduls einen Durchmesser von ca. 8 mm auf, und ein Ärmchen einen Durchmesser von ca. 16 mm auf; bei diesem Beispiel ist das Ärmchen mit dem Durchmesser von ca. 16 mm länger als die beiden anderen Ärmchen und wird in das oben beschriebene Ärmchen des ersten Stentgraft-Moduls mit einem Durchmesser von ca. 14 mm eingeschoben; durch den etwas größeren Durchmesser des Ärmchens des zweiten Stentgraft-Moduls wird durch die radiale Druckwirkung eine feste Verankerung des zweiten Stentgraft-Moduls im ersten Stentgraft-Modul bewirkt.

Es versteht sich, dass auch die Rümpfe der Stentgraft-Module unterschiedliche Durchmesser und Längen aufweisen können. Beispielhaft kann der Durchmesser des Rumpfes des ersten Stentgraft-Moduls einen Durchmesser von zwischen ca. 24 und ca. 36 mm aufweisen, bevorzugt von ca. 32 mm. Die Länge des Rumpfes des ersten Stentgraft-Moduls kann, gemessen vom proximalen, noch von Graftmaterial bedeckten Ende des Rumpfes bis zum distalen Ende des längsten Ärmchens ca. 83 mm betragen.

In einer bevorzugten Ausführungsform kann auch der Rumpf des zweiten Stentgraft-Moduls unterschiedliche Längen und Durchmesser aufweisen; beispielhaft ist der Durchmesser des zweiten Stentgraft-Moduls zwischen ca. 20 und 32 mm, und bevorzugt 28 mm.

Entsprechend weist gemäß einer weiteren Ausführungsform der erste Stentgraft-Modul ein Ärmchen auf, das länger ist und einen größeren Durchmesser aufweist als die beiden anderen Ärmchen. Dabei besitzen in einer Weiterbildung dieser Ausführungsform die beiden anderen Ärmchen des ersten Stentgraft-Moduls einen kleineren aber gleichen Durchmesser und unterscheiden sich aber voneinander in der Länge.

Gemäß einer weiteren Ausführungsform besitzen zwei Ärmchen des zweiten Stentgraft-Moduls die gleiche Länge. Entsprechend ist in einer Weiterbildung bevorzugt, wenn der zweite Stentgraft-Modul ein Ärmchen aufweist, das länger ist und einen größeren Querschnitt aufweist als die zumindest beiden anderen Ärmchen.

Bei einer besonders bevorzugten Weiterbildung der zuvor genannten Ausführungsform sind der erste und der zweite Stentgraft-Modul über ein Ineinanderschieben der jeweils längeren Ärmchen der Stentgraft-Module miteinander verbindbar sind.

In einer weiteren Ausführungsform ist bevorzugt, wenn der erste Stentgraft-Modul an seinem proximalen Ende einen Stent aufweist, der überwiegend von Graftmaterial frei ist.

Diese Ausführungsform hat den Vorteil, dass mit dem vom Graftmaterial freien Bereich eine sichere Verankerung im Gefäß geschaffen werden kann. Die Bögen oder Stützen des Stents legen sich dabei - im Zuge ihrer Selbstexpansion - fest an die Gefäßwand an, so dass in diesem Fall bevorzugt ist, wenn der erste Stentgraft-Modul zuerst gelegt wird, und der zweite Stentgraft-Modul über die Ärmchen mit dem ersten durch Ineinanderverschieben verbunden wird.

Dabei bedeutet "überwiegend frei von Graftmaterial", dass der größte Anteil des Stents nicht von Graftmaterial bedeckt ist, und nur zum Zwecke der Verbindung mit den anderen Stents des Stentgrafts durch das Graftmaterial mit diesen in Verbindung steht. Daher kann es ausreichend sein, wenn nur die äußersten Spitzen der nach distal weisenden Bögen des äußersten Stents mit Graftmaterial bedeckt ist.

Es versteht sich dass der von Prothesen-/Graftmaterial freie Stent am proximalen Ende des ersten Stentgraft-Moduls bzw. die Bögen oder Stützen dieses Stents eine variable Länge aufweisen können, die je nach Platzierungsort und Beschaffenheit des Gefäßes entsprechend angepasst werden kann.

In einer weiteren Ausführungsform ist bevorzugt, wenn zwei Ärmchen des zweiten Stentgraft-Moduls winklig zur röhrenförmigen Längsrichtung des Stentgrafts ausgebildet sind. Der Winkel im Bezug auf die Längsrichtung des Rumpfes des Stentgraft-Moduls kann dabei von ca. 0 bis ca. 45°, insbesondere ca. 30°C, betragen. Diese Ausführungsform hat den Vorteil, dass eine optimale Verbindung zu den anatomisch abgehenden Gefäßabgängen, wie bspw. zu den Arteriae renales, hergestellt werden kann.

Gemäß einer bevorzugten Weiterbildung des erfindungsgemäßen Stentgrafts ist das längere Ärmchen des zweiten Stentgraft-Moduls bezogen auf den Durchmesser des zweiten Rumpfes mittig und die zumindest zwei anderen Ärmchen randständig angeordnet.

Die vorliegende Erfindung betrifft ferner ein Stentgraft-Set, das zumindest zwei erfindungsgemäße modulare Stentgrafts, mithin also mindestens vier Stentgraft-Module, aufweist. Dieses Set bietet den Vorteil, dass es flexibel und in Abhängigkeit des jeweiligen Patienten bzw. dessen Gefäßanatomie zusammengesetzt werden kann; dabei versteht sich, dass bevorzugt ist, wenn die beiden modularen Stentgrafts unterschiedliche Rumpf-Längen und Rumpf-Durchmesser besitzen, um ein noch flexibleres Einsetzen zu ermöglichen. Dieses Set ist insbesondere bei Notfällen und Notoperationen vorteilhaft, bei welchen ein schnelles Handeln notwendig ist, und eine spezifische Anpassung/Ausmessung und Vorgabe für einen maßgeschneiderten Stentgraft mangels Zeitnot nicht möglich ist.

Die vorliegenden Offenbarung betrifft ferner auch ein Verfahren zur Einführung eines wie zuvor beschriebenen, erfindungsgemäßen modularen Stentgrafts, in ein Blutgefäß eines Patienten bzw. ein Verfahren zur Behandlung eines Patienten, der der Einführung oder Legung eines Stentgrafts bedarf, mit dem folgenden aufeinanderfolgenden Schritten:
- Einbringen des ersten Stentgraft-Moduls in das zu behandelnde Blutgefäß,
- expandierendes Freisetzen des ersten Stentgraft-Moduls an einer zu behandelnden Stelle in dem Blutgefäß;
- Einbringen des zweiten Stentgraft-Moduls in das gleiche zu behandelnde Blutgefäß, und
- Teilweises Einführen eines Ärmchens des zweiten Stentgraft-Moduls in ein Ärmchen des ersten Stentgraft-Moduls und expandierendes Freisetzen des zweiten Stentgraft-Moduls zur teilweisen Überlappung des distalen Endes des Ärmchens des ersten Stentgraft-Moduls mit dem proximalen Ende des Ärmchens des zweiten Stentgraft-Moduls.

Die Einführung und Freisetzung der Stentgraft-Module kann dabei mit im Stand der Technik bekannten Einführ- und Freisetzungssystemen erfolgen; beispielhaft wird hierbei auf die in den DE 10 346 200, DE 10 2006 004123 und DE 10 2007 010 305 beschriebenen Systeme verwiesen.

Alternativ können die Stentgraft-Module auch in umgekehrter Reihenfolge eingeführt werden, wobei das Verfahren dann die folgenden Schritte umfasst:
- Einbringen des zweiten Stentgraft-Moduls in das zu behandelnde Blutgefäß,
- expandierendes Freisetzen des zweiten Stentgraft-Moduls an einer zu behandelnden Stelle in dem Blutgefäß;
- Einbringen des ersten Stentgraft-Moduls in das gleiche zu behandelnde Blutgefäß, und
- Teilweises Einführen eines Ärmchens des ersten Stentgraft-Moduls in ein Ärmchen des zweiten Stentgraft-Moduls und expandierendes Freisetzen des ersten Stentgraft-Moduls zur teilweisen Überlappung des distalen Endes des Ärmchens des zweiten Stentgraft-Moduls mit dem proximalen Ende des Ärmchens des ersten Stentgraft-Moduls.

In bevorzugten Ausführungsformen des Verfahrens werden die weiter oben beschriebenen vorteilhaften Weiterbildungen des erfindungsgemäßen modularen Stentgrafts eingesetzt, mithin also bspw. die jeweils längsten Ärmchen der beiden Stentgraft-Module ineinander eingeführt.

Die Offenbarung betrifft ferner die Verwendung eines erfindungsgemäßen modularen Stentgrafts zur Behandlung eines Blutgefäßes, wobei beide Module des modularen Stentgrafts in das gleiche Blutgefäß eingeführt werden.

Weitere Vorteile ergeben sich aus der Beschreibung und den beigefügten Figuren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung wie beansprucht zu verlassen.

Ausführungsbeispiele der Erfindung sind in den Zeichnungen dargestellt und werden in der nachstehenden Beschreibung näher erläutert. Es zeigen:
Fig. 1 eine schematische Darstellung eines Ausführungsbeispiels für einen ersten Stentgraft-Modul des erfindungsgemäßen Stentgrafts in seitlicher Ansicht und mit Graftmaterial (A); sowie schematisch von vorne ohne Graftmaterial (B);
Fig. 2 eine schematische Darstellung eines Ausführungsbeispiels für einen zweiten Stentgraft-Modul des erfindungsgemäßen Stentgrafts in seitlicher Ansicht und mit Graftmaterial (A); sowie schematisch von vorne ohne Graftmaterial (B);
Fig. 3 eine schematische Darstellung eines weiteren Ausführungsbeispiels für einen zweiten Stentgraft-Modul des erfindungsgemäßen Stentgrafts in seitlicher Ansicht und mit Graftmaterial (A); sowie schematisch von vorne ohne Graftmaterial (B); und
Fig. 4 eine schematische Darstellung einer Ausführungsform des modularen Stentgrafts, zusammengesetzt aus den in Fig. 1 und 2 gezeigten ersten und zweiten Stentgraft-Modulen, in seitlicher Ansicht und mit Graftmaterial.

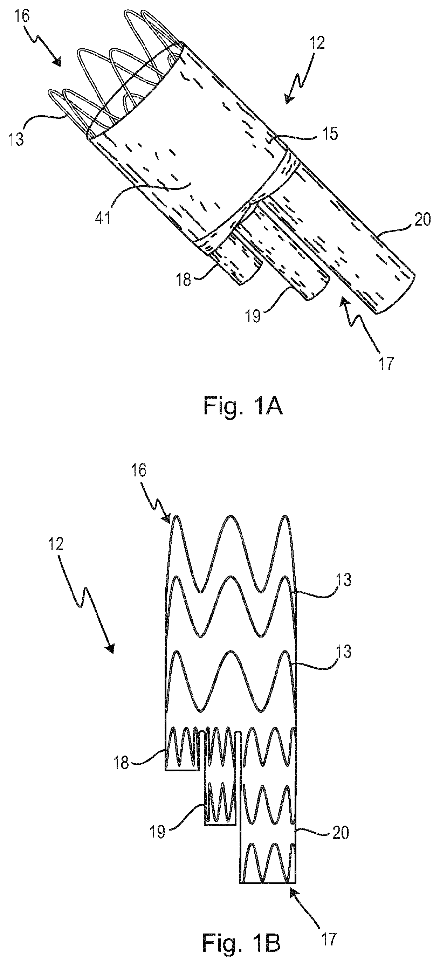

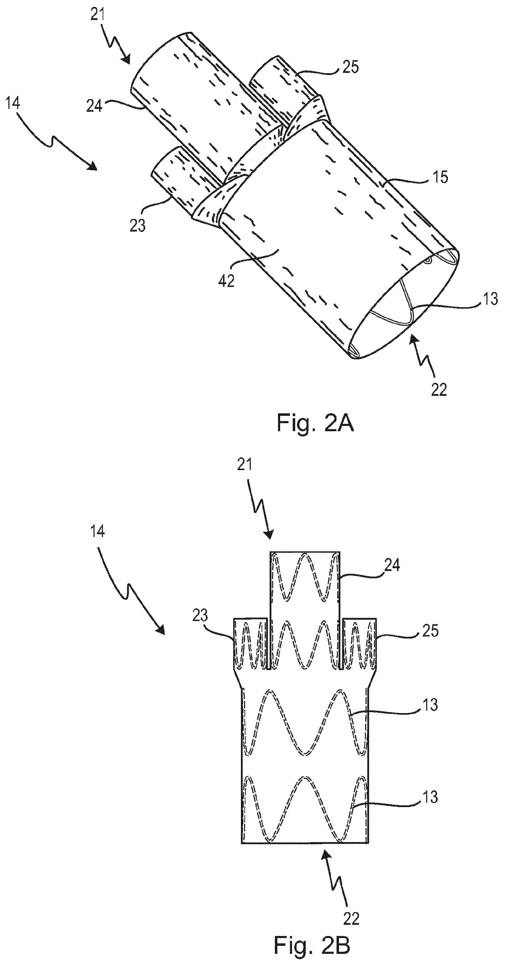

In Fig. 1 A und B ist mit 12 ein erster Stentgraft-Modul bezeichnet; sowie in Fig. 2 A und B mit 14 ein zweiter Stentgraft-Modul. Beide Stentgraft-Module 12, 14 weisen jeweils mehrere Stents 13 auf, die mäanderförmig umlaufende Stützen mit abwechselnd in proximale und distale Richtung weisende Bögen aufweisen. Die Stents 13 werden auch als "Stentringe" bezeichnet. Die Stents 13 der Stentgraft-Module 12, 14, welche vorzugsweise aus einem selbstexpandierenden Material mit Shape-Memory Eigenschaft, vorzugsweise Nitinol, sind, sind jeweils über ein biokompatibles Graftmaterial 15 verbunden; dieses ist in den Figuren 1 B, 2B und 3B lediglich zu Übersichtszwecken durchsichtig gezeichnet. Die Stentgraft-Module 12, 14 weisen vorzugsweise das gleiche Material 15 auf, das aus üblicherweise für Stentgrafts verwendeten Materialien besteht. Dem Fachmann wird ausgehend von der hierin offenbarten Lehre klar sein, welche Materialien in Frage kommen, um den erfindungsgemäßen Stentgraft umzusetzen.

Der in Fig. 1A und 1 B gezeigte Stentgraft-Modul, der einen Rumpf 41, ein proximales Ende 16 und ein distales Ende 17 besitzt, weist ferner am distalen Ende 17 drei Ärmchen 18, 19, 20 auf, die sich in distale Richtung erstrecken. Das Ärmchen 20 weist dabei einen größeren Durchmesser auf als die beiden anderen Ärmchen 18 und 19, und ist ferner auch länger als diese.

Auch der in Fig. 2A und 2B gezeigte Stentgraft-Modul 14, der einen Rumpf 42, ein proximales Ende 21 und ein distales Ende 22 besitzt, weist am proximalen Ende 21 drei Ärmchen 23, 24 und 25 auf. Das Ärmchen 24 ist in der in Fig. 2 gezeigten Ausführungsform des zweiten Stentgraft-Moduls 14 länger als die beiden anderen Ärmchen 24 und 25 und besitzt auch einen größeren Durchmesser.

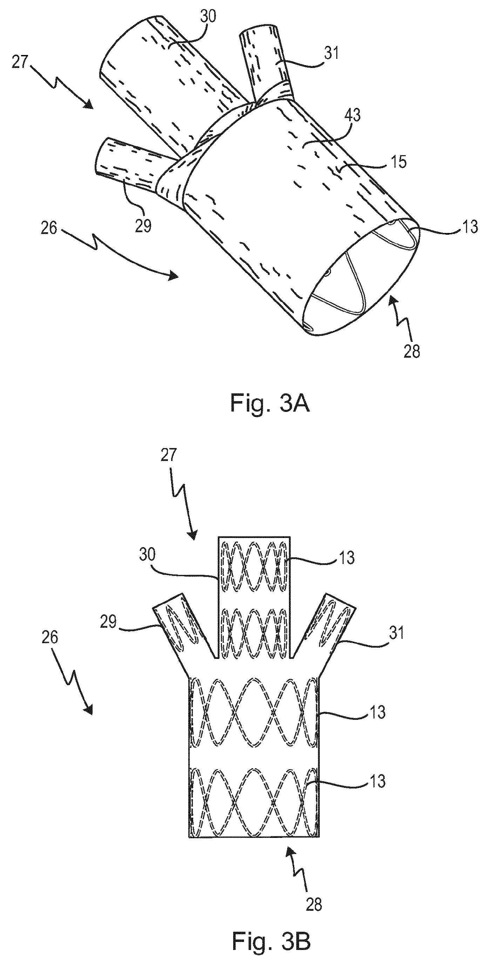

In Fig. 3 ist eine alternative, weitere Ausführungsform des zweiten Stentgraft-Moduls 26 gezeigt, mit einem Rumpf 43, einem proximalen Ende 27 und einem distalen Ende 28. Auch dieser Stentgraft-Modul 26 weist drei Ärmchen 29, 30 und 31 auf, wobei auch hier das längere Ärmchen 30 einen größeren Durchmesser als die beiden anderen Ärmchen 29 und 31 besitzt.

Bei der in der Fig. 3 gezeigten Ausführungsform erstreckt sich das Ärmchen 30 parallel zur Längsrichtung des Rumpfes 43, wohingegen die beiden Ärmchen 29 und 31 winklig in Bezug auf die Längsrichtung abstehen. Der Winkel, mit dem die beiden Ärmchen 29 und 31 abstehen, kann dabei zwischen 0° und ca. 30° betragen.

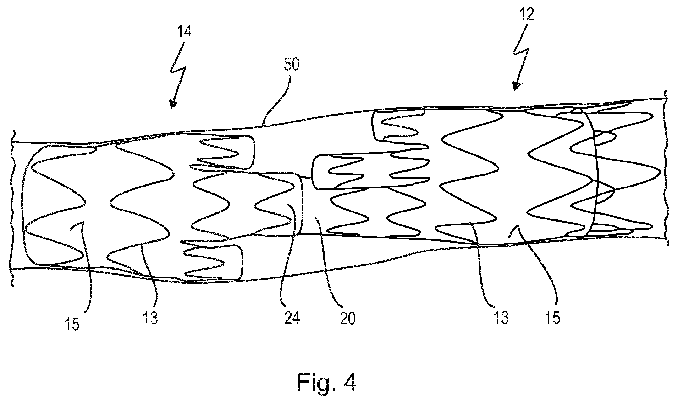

In Fig. 4 ist eine Ausführungsform des erfindungsgemäßen modularen Stentgrafts 10 gezeigt, der aus den beiden Stentgraft-Modulen 12 und 14 zusammengesetzt ist. In der in Fig. 4 gezeigten Darstellung ist das Ärmchen 20 des ersten Stentgraft-Moduls 12 in das Ärmchen 24 des zweiten Stentgraft-Moduls 14 derart teilweise eingeführt, dass das proximale Ende des Ärmchens 24 mit dem distalen Ende des Ärmchens 20 überlappt. Bei dieser Ausführungsform ist daher der Durchmesser des Ärmchens 20 vorzugsweise größer als der Durchmesser des Ärmchens 24.

Es versteht sich, dass alternativ auch das Ärmchen 24 des zweiten Stentgraft-Moduls 14 in das Ärmchen 20 des ersten Stentgraft-Moduls 12 teilweise überlappend eingeführt sein kann; in diesem Fall ist vorzugsweise der Durchmesser des Ärmchens 24 größer als der Durchmesser des Ärmchens 20 des ersten Stentgraft-Moduls, um nach der Expansion des Moduls und des Ärmchens einen festen Druck und dadurch eine Verankerung des Ärmchens 24 im Ärmchen 20 zu gewährleisten.

Der modulare Stentgraft 10 ist in der in Fig. 4 gezeigten Darstellung in einem Blutgefäß 50 eingeführt; auch hieraus ist zu erkennen, dass der Stentgraft 10 nicht an /wegen einer Verzweigung modulartig zusammengebaut ist, sondern sich die Module 12 und 14 im gleichen Gefäß 50 befinden, und nicht in abzweigenden Blutgefäßen vorliegen.

## Patentansprüche

1. Modularer Stentgraft (10), der zur Einbringung in ein Blutgefäß (50) eines Patienten ausgebildet ist, und der über ein Graftmaterial (15) verbundene Stents (13) aufweist, **dadurch gekennzeichnet, dass** der Stentgraft (10)
- einen ersten Stentgraft-Modul (12) aufweist, mit einem Rumpf (41), einem proximalen (16) und einem distalen (17) Ende, sowie mit zumindest zwei sich in Längsrichtung vom distalen Ende (17) des ersten Stentgraft-Moduls (12) aus in distale Richtung erstreckenden röhrenförmigen Ärmchen (18, 19, 20), die einstückig mit dem Rumpf (41) des ersten Stentgraft-Moduls (12) verbunden sind,
- einen zweiten Stentgraft-Modul (14; 26), mit einem zweiten Rumpf (42; 43), einem proximalen (21; 27)) und einem distalen (22; 28)) Ende, sowie mit zumindest drei sich in Längsrichtung vom proximalen Ende (21; 28) des zweiten Stentgraft-Moduls (14; 26) in proximale Richtung erstreckenden röhrenförmigen Ärmchen (23, 24, 25; 29, 30, 31), die einstückig mit dem Rumpf (42; 43) des zweiten Stentgraft-Moduls (14; 26) ausgebildet sind,
wobei jeweils ein Ärmchen (20; 24; 30) des ersten (12) und des zweiten (14; 26) Stentgraft-Moduls Durchmesser aufweisen, derart, dass der erste und zweite Stentgraft-Modul (12; 14; 26) durch ein Ineinanderschieben und durch teilweises Überlappen des jeweils einen Ärmchens (20) des distalen Endes (17) des ersten Stentgraft-Moduls (12) und des Ärmchens (24; 30) des proximalen Endes (21; 27) des zweiten Stentgraft-Moduls (14; 26) zur Ausbildung eines zusammengesetzten modularen Stentgrafts (10) im gleichen Blutgefäß (50) miteinander verbindbar sind.

2. Stentgraft (10) nach Anspruch 1, **dadurch gekennzeichnet, dass** der erste (12) und der zweite Stentgraft-Modul (14; 26) durch Ineinanderschieben eines Ärmchens (24; 30) des zweiten Stentgraft-Moduls (14; 26) in ein Ärmchen (20) des ersten Stentgraft-Moduls (12) und durch deren teilweises Überlappen verbindbar sind.

3. Stentgraft (10) nach Anspruch 1, **dadurch gekennzeichnet, dass** der erste (12) und der zweite Stentgraft-Modul (14) durch Ineinanderschieben eines Ärmchens (20) des ersten Stentgraft-Moduls (12) in ein Ärmchen (24; 30) des zweiten Stentgraft-Moduls (14; 26) und durch deren teilweises Überlappen verbindbar sind.

4. Stentgraft (10) nach einem der vorstehenden Ansprüche, **dadurch gekennzeichnet, dass** der erste Stentgraftmodul (12) zwischen zwei und vier, insbesondere drei Ärmchen (18, 19, 20) aufweist.

5. Stentgraft (10) nach einem der Ansprüche 1 bis 4, **dadurch gekennzeichnet, dass** die Ärmchen (18, 19, 20; 23, 24, 25; 29, 30, 31) des ersten Stentgraft-Moduls (12) und/oder des zweiten Stentgraft-Moduls (14) unterschiedliche Längen und/oder unterschiedliche Durchmesser aufweisen.

6. Stentgraft (10) nach einem der Ansprüche 1 bis 5, **dadurch gekennzeichnet, dass** der erste Stentgraft-Modul (12) ein Ärmchen (20) aufweist, das länger ist und einen größeren Durchmesser aufweist als das zumindest zweite andere Ärmchen (18, 19).

7. Stentgraft (10) nach einem der Ansprüche 1 bis 6, **dadurch gekennzeichnet, dass** zumindest zwei Ärmchen (23, 25; 29, 31) des zweiten Stentgraft-Moduls (14; 26) die gleiche Länge aufweisen.

8. Stentgraft (10) nach einem der Ansprüche 1 bis 7, **dadurch gekennzeichnet, dass** der zweite Stentgraft-Modul (14; 26) ein Ärmchen (24; 30) aufweist, das länger ist und einen größeren Querschnitt aufweist als die zumindest beiden anderen Ärmchen (23, 25; 29, 31).

9. Stentgraft (10) nach Anspruch 8 und 4, **dadurch gekennzeichnet, dass** der erste (12) und der zweite (14) Stentgraft-Modul über ein Ineinanderschieben der jeweils längeren Ärmchen (20; 24; 30) der Stentgraft-Module (12, 14) miteinander verbindbar sind.

10. Stentgraft (10) nach einem der Ansprüche 1 bis 9, **dadurch gekennzeichnet, dass** der erste Stentgraft-Modul (12) an seinem proximalen Ende (16) einen Stent (13) aufweist, der überwiegend von Graftmaterial (15) frei ist.

11. Stentgraft (10) nach einem der Ansprüche 1 bis 10, **dadurch gekennzeichnet, dass** zumindest zwei Ärmchen (29, 31) des zweiten Stentgraft-Moduls (26) winklig zur röhrenförmigen Längsrichtung des zweiten Stentgraft-Moduls (26) ausgebildet sind.

12. Stentgraft (10) nach einem der Ansprüche 8 bis 11, **dadurch gekennzeichnet, dass** das längere Ärmchen (24; 30) des zweiten Stentgraft-Moduls (14; 26) bezogen auf den Durchmesser des zweiten Rumpfes (42; 43) mittig und die zumindest zwei anderen Ärmchen (23, 25; 29, 31) randständig angeordnet sind.

13. Stentgraft-Set, umfassend zumindest zwei modulare Stentgrafts (10) nach einem der Ansprüche 1 bis 12.

## Claims

1. A modular stent graft (10) which is designed for introduction into a blood vessel (50) of a patient and which has stents (13) connected by means of a graft material (15), **characterized in that** the stent graft (10) comprises
- a first stent graft module (12) with a body (41), a proximal end (16) and a distal end (17), and with at least two tubular arms (18, 19, 20) which extend longitudinally in the distal direction from the distal end (17) of the first stent graft module (12) and which are integrally connected to the body (41) of the first stent graft module (12),
- a second stent graft module (14; 26) with a second body (42; 43), a proximal end (21; 27) and a distal end (22; 28), and with at least three tubular arms (23, 24, 25; 29, 30, 31) which extend longitudinally in the proximal direction from the proximal end (21; 28) of the second stent graft module (14; 26) and which are integrally formed with the body (42; 43) of the second stent graft module (14; 26),
wherein arms (20; 24; 30) of the first stent graft module (12) and of the second stent graft module (14; 26) in each case have diameters such that the first and second stent graft modules (12; 14; 26) are connectable to each other by being pushed into each other and by partially overlapping the respective one arm (20) of the distal end (17) of the first stent graft module (12) with the arm (24; 30) of the proximal end (21; 27) of the second stent graft module (14; 26) in order to form an assembled modular stent graft (10) within the same blood vessel (50).

2. The stent graft (10) as claimed in claim 1, **characterized in that** the first stent graft module (12) and the second stent graft module (14; 26) are connectable by introducing an arm (24; 30) of the second stent graft module (14; 26) into an arm (20) of the first stent graft module (12) and by having them partially overlap.

3. The stent graft (10) as claimed in claim 1, **characterized in that** the first stent graft module (12) and the second stent graft module (14) are connectable by introducing an arm (20) of the first stent graft module (12) into an arm (24; 30) of the second stent graft module (14; 26) and by having them partially overlap.

4. The stent graft (10) as claimed in one of the preceding claims, **characterized in that** the first stent graft module (12) has between two and four arms, in particular three arms (18, 19, 20).

5. The stent graft (10) as claimed in one of claims 1 through 4, **characterized in that** the arms (18, 19, 20; 23, 24, 25; 29, 30, 31) of the first stent graft module (12) and/or of the second stent graft module (14) have different lengths and/or different diameters.

6. The stent graft (10) as claimed in one of claims 1 through 5, **characterized in that** the first stent graft module (12) has one arm (20) longer than and with a larger diameter than the at least second other arm (18, 19).

7. The stent graft (10) as claimed in one of claims 1 through 6, **characterized in that** at least two arms (23, 25; 29, 31) of the second stent graft module (14; 26) have the same length.

8. The stent graft (10) as claimed in one of claims 1 through 7, **characterized in that** the second stent graft module (14; 26) has one arm (24; 30) longer than and with a larger cross section than the at least two other arms (23, 25; 29, 31).

9. The stent graft (10) as claimed in claims 8 and 4, **characterized in that** the first stent graft module (12) and the second stent graft module (14) are connectable to each other by means of the respective longer arms (20; 24; 30) of the stent graft modules (12, 14) being pushed into each other.

10. The stent graft (10) as claimed in one of claims 1 through 9, **characterized in that** the first stent graft module (12) has, at its proximal end (16), a stent (13) that is largely free of graft material (15).

11. The stent graft (10) as claimed in one of claims 1 through 10, **characterized in that** at least two arms (29, 31) of the second stent graft module (26) are formed at an angle to the tubular longitudinal direction of the second stent graft module (26).

12. The stent graft (10) as claimed in one of claims 8 through 11, **characterized in that**, with respect to the diameter of the second body (42; 43), the longer arm (24; 30) of the second stent graft module (14; 26) is arranged centrally and the at least two other arms (23, 25; 29, 31) are arranged at the edges.

13. A stent graft kit comprising at least two modular stent grafts (10) as claimed in one of claims 1 through 12.

## Revendications

1. Endoprothèse modulaire (10), qui est destinée à être introduite dans un vaisseau sanguin (50) d'un patient, et qui présente des stents (13) assemblés par un matériau de greffe (15), **caractérisée en ce que** l'endoprothèse (10)
- présente un premier module d'endoprothèse (12), avec un tronc (41), une extrémité proximale (16) et une extrémité distale (17), ainsi qu'avec au moins deux branches tubulaires (18, 19, 20) s'étendant en direction longitudinale depuis l'extrémité distale (17) du premier module d'endoprothèse (12) en direction distale, qui sont connectées d'une seule pièce au tronc (41) du premier module d'endoprothèse (12), et
- présente un deuxième module d'endoprothèse (14; 26), avec un deuxième tronc (42; 43), une extrémité proximale (21; 27) et une extrémité distale (22; 28), ainsi qu'avec au moins trois branches tubulaires (23, 24, 25; 29, 30, 31) s'étendant en direction longitudinale depuis l'extrémité proximale (21; 28) du deuxième module d'endoprothèse (14; 26) en direction proximale, qui sont formées d'une seule pièce avec le tronc (42; 43) du deuxième module d'endoprothèse (14; 26),
dans laquelle une branche (20; 24; 30) du premier (12) et du deuxième (14; 26) modules d'endoprothèse présente respectivement des diamètres, tels que le premier et le deuxième modules d'endoprothèse (12; 14; 26) puissent être assemblés l'un à l'autre par insertion l'une dans l'autre et par chevauchement partiel respectivement d'une branche (20) de l'extrémité distale (17) du premier module d'endoprothèse (12) et de la branche (24; 30) de l'extrémité proximale (21; 27) du deuxième module d'endoprothèse (14; 26) pour former une endoprothèse modulaire composée (10) dans le même vaisseau sanguin (50).

2. Endoprothèse (10) selon la revendication 1, **caractérisée en ce que** le premier (12) et le deuxième (14; 26) modules d'endoprothèse peuvent être assemblés par insertion l'une dans l'autre d'une branche (24; 30) du deuxième module d'endoprothèse (14; 26) dans une branche (20) du premier module d'endoprothèse (12) et par leur chevauchement partiel.

3. Endoprothèse (10) selon la revendication 1, **caractérisée en ce que** le premier (12) et le deuxième (14) modules d'endoprothèse peuvent être assemblés par insertion l'une dans l'autre d'une branche (20) du premier module d'endoprothèse (12) dans une branche (24; 30) du deuxième module d'endoprothèse (14; 26) et par leur chevauchement partiel.

4. Endoprothèse (10) selon l'une quelconque des revendications précédentes, **caractérisée en ce que** le premier module d'endoprothèse (12) présente entre deux et quatre, en particulier trois branches (18, 19, 20).

5. Endoprothèse (10) selon l'une quelconque des revendications 1 à 4, **caractérisée en ce que** les branches (18, 19, 20; 23, 24, 25; 29, 30, 31) du premier module d'endoprothèse (12) et/ou du deuxième module d'endoprothèse (14) présentent des longueurs différentes et/ou des diamètres différents.

6. Endoprothèse (10) selon l'une quelconque des revendications 1 à 5, **caractérisée en ce que** le premier module d'endoprothèse (12) présente une branche (20), qui est plus longue et qui présente un plus grand diamètre que ladite au moins une deuxième autre branche (18, 19).

7. Endoprothèse (10) selon l'une quelconque des revendications 1 à 6, **caractérisée en ce qu'**au moins deux branches (23, 25; 29, 31) du deuxième module d'endoprothèse (14; 26) présentent la même longueur.

8. Endoprothèse (10) selon l'une quelconque des revendications 1 à 7, **caractérisée en ce que** le deuxième module d'endoprothèse (14; 26) présente une branche (24; 30), qui est plus longue et qui présente une plus grande section transversale que lesdites au moins deux autres branches (23, 25; 29, 31).

9. Endoprothèse (10) selon la revendication 8 et 4, **caractérisée en ce que** le premier (12) et le deuxième (14) modules d'endoprothèse peuvent être assemblés l'un à l'autre par insertion l'une dans l'autre de la branche respectivement plus longue (20; 24; 30) des modules d'endoprothèse (12, 14).

10. Endoprothèse (10) selon l'une quelconque des revendications 1 à 9, **caractérisée en ce que** le premier module d'endoprothèse (12) présente à son extrémité proximale (16) un stent (13), qui est essentiellement libre de matériau de greffe (15).

11. Endoprothèse (10) selon l'une quelconque des revendications 1 à 10, **caractérisée en ce qu'**au moins deux branches (29, 31) du deuxième module d'endoprothèse (26) forment un angle avec la direction longitudinale tubulaire du deuxième module d'endoprothèse (26).

12. Endoprothèse (10) selon l'une quelconque des revendications 8 à 11, **caractérisée en ce que** la branche plus longue (24; 30) du deuxième module d'endoprothèse (14; 26) est disposée au milieu et lesdites au moins deux autres branches (23, 25; 29, 31) sont disposées sur le bord par rapport au diamètre du deuxième tronc (42; 43).

13. Ensemble d'endoprothèse, comprenant au moins deux endoprothèses modulaires (10) selon l'une quelconque des revendications 1 à 12.
